# EUROPEAN PATENT APPLICATION

(11) **EP 3 581 194 A1**
(43) Date of publication of application: **18.12.2019**
(21) Application number: 19180059.8
(22) Date of filing: 13.06.2019
(51) Int. Cl.: A61K 38/00

(54) **PROTEIN KINASE INHIBITORS AND THEIR USES IN THE TREATMENT OF CANCER**

(30) Priority: 13.06.2018 US 201816007146
(71) Applicant: King Faisal Specialist Hospital & Research Centre, 11211 Riyadh (SA)
(72) Inventor: Abu Khabar, Khalid S., 11211 Riyadh (SA); Al-Qahtani, Qamraa Hamad, 11211 Riyadh (SA)
(74) Representative: Engelhard, Markus

(57) **Abstract**

The present invention relates to protein kinase inhibitors for use in a method of treatment of cancer, said method comprising administering an effective dose of a protein kinase inhibitor to a patient in need thereof having said cancer. The present invention also relates to protein kinase inhibitors for use in a method of post-transcriptional control of cancer-related genes comprising administering an effective amount of a protein kinase inhibitor to a subject in need thereof.

## Description

### FIELD OF THE INVENTION

The present invention relates to protein kinase inhibitors for use in a method of treatment of cancer, said method comprising administering an effective dose of a protein kinase inhibitor to a patient in need thereof having said cancer. The present invention also relates to protein kinase inhibitors for use in a method of post-transcriptional control of cancer-related genes comprising administering an effective amount of a protein kinase inhibitor to a subject in need thereof.

### BACKGROUND OF THE INVENTION

Cancers are a diverse variety of pathological conditions. One example is breast cancer (BC) which is the most common form of female malignancies, representing a major cause of death from cancer among the women worldwide. Breast cancer is characterized by alteration in the expression of many genes involved in cell cycle, growth and differentiation, DNA repair, apoptosis, inflammation, angiogenesis, invasiveness, and metastasis.

Gene expression is regulated by different mechanisms, including transcriptional, post-transcriptional, and post-translational modification mechanisms. Post-transcriptional control represents an essential level of gene expression fine tuning and comprises processes such as mRNA decay, mRNA transport, and translation. mRNA decay affects the level of available mRNA for translation and it is a tightly regulated process that mainly relies on the presence of a *cis*-acting sequence in the primary transcript of the mRNA to which *trans*-acting proteins bind and confer stability or instability of the mRNA.

Among the well-known and extensively studied *cis*-acting mRNA instability determinants are adenylate-uridylate-rich elements (AU-rich elements, AREs). Several ARE binding proteins (ARE-BP) are involved in the pathogenesis of cancer. Specifically, many human tumors are found be associated with deficiency of tristetraprolin (TTP, ZFP36) and/or overexpression of human antigen R (HuR). The aberrant expression of these proteins can derive from misregulation on various regulational levels including transcriptional regulation, epigenetic regulation, post-transcriptional regulation, and post-translational regulation.

Phosphorylation of ARE-BPs by different protein kinases is a mechanism of post-translational modification that highly affects the cellular localization and activity of said ARE-BPs. Protein phosphorylation results in alteration of protein structure and conformation, and modifies its activity and function. The commonly phosphorylated amino acids in eukaryotes are serine, threonine, and tyrosine. The phosphorylation is mediated through the action of a protein kinase (PK), and can be reverse through the action of a phosphatase. Nearly 2% of the human genome encode for PKs, representing about 538 genes which are subdivided into typical, or conventional, and atypical protein kinases, according to the kinase database (http://kinase.com/kinbase/). The majority of typical PKs phosphorylates serine/threonine (STPKs) and only a minority of PKs phosphorylates tyrosine, and atypical PKs are mostly STPKs. To date, FDA has approved 37 small molecule kinase inhibitors and many others are in phase-2/3 clinical trials. Most of the approved kinase drugs are intended for treatment of cancers, and only few of them have been approved for treatment of non-cancerous conditions, such as sirolimus for organ rejection.

Polo-like kinases (PLKs) are a family of regulatory serine/threonine kinases comprising five members including polo-like kinase 1 (PLK-1), as well as PLK-2, PLK-3, PLK-4, and PLK-5. Polo-like kinases are involved in the cell cycle at various stages, including mitosis, spindle formation, cytokinesis, and meiosis. Beyond cell cycle regulation, there is evidence that PLKs play regulatory roles in different cellular pathways and an increasing amount of PLK substrates is revealed. For example, PLK-1 has been found to phosphorylate insulin receptor substrate (IRS), β-catenin, heat-shock protein 70, mTOR, vimentin, and the breast cancer susceptibility protein (BRCA2).

Regulating aberrant expression of cancer-related genes using ARE-BPs is a potential approach for a method of treatment of cancer.

US 2010/0055705 A1 discloses compositions and methods for diagnosing and treating cancer, including TTP as a biomarker and therapeutic option for the treatment of cancer.

EP 2 435 041 B1 relates to a therapeutic combination comprising a PLK-1 inhibitor and an antineoplastic agent.

Bhola et al. [1] disclose a kinome-wide functional screen identifying a role of PLK-1 in acquired hormone-independent growth of ER⁺ human breast cancer.

Maire et al. [2] relates to a PLK-1 inhibitor as potential therapeutic option for the management of patients with triple-negative breast cancer.

However, a method of treatment of cancer involving post-transcriptional control of expression of cancer-related genes, comprising administering a protein kinase inhibitor for normalizing the levels of TTP and HuR, has not been described. Thus, the present invention aims at a method of treatment of cancer, wherein said cancer is characterized by aberrant expression of cancer-related genes and/or ARE-BPs.

The present inventors have used a commercially available kinase inhibitor library that comprises 378 drugs comprising FDA approved agents. High-throughput screening was performed using said library by conducting an optimized highly selective post-transcriptional reporter assay that was designed to identify hits affecting ARE-mediated post-transcriptional regulation. Compounds from the PK inhibitor library were scored as hits if they reduced the expression of ARE-containing reporter activity compared to control reporter activity. The present inventors disclose a method of treatment of cancer using ARE-mediated post-transcriptional regulation of gene expression involving administering a protein kinase inhibitor, namely a B-Raf kinase inhibitor, VEGFR2 inhibitor, or a polo-like kinase inhibitor, preferably a polo-like kinase 1 inhibitor, to a patient in need thereof.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a protein kinase inhibitor for use in a method of treatment of cancer in a patient, wherein said cancer is characterized by one of the following:
underexpression of TTP and overexpression of HuR,
underexpression of TTP and overexpression of PLK-1,
overexpression of HuR and overexpression of PLK-1,
underexpression of TTP and overexpression of HuR and overexpression of PLK-1,
in cancer cells compared to expression in non-cancerous cells;
said method comprising administering an effective dose of a protein kinase inhibitor to a patient in need thereof having said cancer, wherein said protein kinase inhibitor is a B-Raf kinase inhibitor, VEGFR2 inhibitor, or polo-like kinase inhibitor, preferably a polo-like kinase 1 inhibitor.

In one embodiment, said method comprises the steps of:
a. Receiving a sample of a tumor (tumor sample), and optionally a control sample, from the patient,
b. Determining the level of expression of TTP, and/or HuR, and/or PLK-1 in said tumor sample, and optionally in said control sample,
c. Administering a therapeutically effective amount of said protein kinase inhibitor, preferably of said polo-like kinase inhibitor (PLK), more preferably a PLK-1 inhibitor to the patient, if there is a reduced expression of TTP and/or increased expression of HuR, and/or increased expression of PLK-1 in the tumor sample as compared to a control sample, which is optionally the control sample of said patient, as determined in step b).

In one embodiment, said cancer comprises cells having diminished levels of TTP and/or elevated levels of HuR compared to normal cells.

In one embodiment, said cancer is invasive breast cancer.

In one embodiment, said cancer is triple-negative breast cancer.

In one embodiment, said protein kinase inhibitor is selected from the group comprising AZ628, sorafenib2, TAK-6323, regorafenib4, CEP-32496, cabozantinib, and polo-like kinase inhibitors including volasertib.

In one embodiment, said protein kinase inhibitor is a polo-like kinase inhibitor, preferably a polo-like kinase 1 inhibitor, preferably a specific polo-like kinase 1 inhibitor.

In one embodiment, said polo-like kinase inhibitor is selected from the group comprising PCM-075, volasertib, BI 2536, rigosertib (ON 01910), HMN-214, GSK461364, Ro3280, NMS-P937, TAK-960, cyclapolin 1, DAP-81, ZK-thiazolidinone, compound 36 (imidazopyridine derivative), LFM-A13, poloxin (thymoquinone derivative), poloxipan, purpurogallin (benzotropolone-containing compound), MLN0905, SBE13.

In one embodiment, said polo-like kinase inhibitor is a polo-like kinase 1 inhibitor.

In one embodiment, said polo-like kinase inhibitor is preferably a dihydropteridinone-based derivative, and more preferably volasertib.

In one embodiment, said protein kinase inhibitor is co-administered with a chemotherapeutic agent, and/or a checkpoint inhibitor, and/or an interferon selected from Type-I IFN, Type-II IFN and Type-III IFN.

In one embodiment, said checkpoint inhibitor is selected from CTLA-4, PD-1, and PD-L1 targeting agents.

In one embodiment, said checkpoint inhibitor is selected from the group comprising ipilimumab, tremelimumab, nivolumab, MK-3475, MPDL-3280A, MEDI-4736, and BMS-936559.

In one embodiment, said interferon, preferably said Type-I IFN and/or said Type-II IFN and/or said Type-III IFN, enhances TTP expression and/or reduces HuR expression, wherein preferably said protein kinase inhibitor and said interferon synergistically enhance TTP expression and/or reduce HuR expression.

In one embodiment, said TTP expression is increased and/or HuR expression is decreased by administering said protein kinase inhibitor.

In one embodiment, cancer-related genes are post-transcriptionally controlled by administering said protein kinase inhibitor.

In a further aspect, the present invention relates to a protein kinase inhibitor for use in a method of post-transcriptional control of cancer-related genes comprising administering an effective amount of a protein kinase inhibitor to a subject in need thereof, wherein said protein kinase inhibitor is a B-Raf kinase inhibitor, VEGFR2 inhibitor, or polo-like kinase inhibitor, preferably a polo-like kinase 1 inhibitor.

In one embodiment, said administering of a protein kinase inhibitor results in the reduction of expression of a mRNA comprising an AU-rich element.

In one embodiment, said protein kinase inhibitor is selected from inhibitors of polo-like kinase 1, including AZ628, sorafenib2, TAK-6323, regorafenib4, CEP-32496, cabozantinib, PCM-075, volasertib, BI 2536, rigosertib (ON 01910), HMN-214, GSK461364, Ro3280, NMS-P937, TAK-960, cyclapolin 1, DAP-81, ZK-thiazolidinone, compound 36 (imidazopyridine derivative), LFM-A13, poloxin (thymoquinone derivative), poloxipan, purpurogallin (benzotropolone-containing compound), MLN0905, SBE13, wherein said protein kinase inhibitor is preferably a polo-like kinase inhibitor, more preferably a polo-like kinase 1 inhibitor, and more preferably volasertib.

In one embodiment, said cancer, said protein kinase inhibitor and said administering are as defined above.

In a further aspect, the present invention relates to a protein kinase inhibitor for use in a method of treatment of cancer, wherein said cancer is characterized by one of the following:
underexpression of TTP and overexpression of HuR,
underexpression of TTP and overexpression of PLK-1,
overexpression of HuR and overexpression of PLK-1,
underexpression of TTP and overexpression of HuR and overexpression of PLK-1,
in cancer cells compared to expression in non-cancerous cells;
said method comprising administering an effective dose of a protein kinase inhibitor, to a patient in need thereof having said cancer, wherein said protein kinase inhibitor is a B-Raf kinase inhibitor, VEGFR2 inhibitor, or polo-like kinase inhibitor, preferably a polo-like kinase 1 inhibitor.

In one embodiment, said protein kinase inhibitor is co-administered with a chemotherapeutic agent, and/or a checkpoint inhibitor, and/or Type-I IFN.

Said method, said cancer, said protein kinase inhibitor, said polo-like kinase inhibitor, said polo-like kinase 1 inhibitor, said co-administered, said checkpoint inhibitor, said Type-I IFN are as defined above.

In a further aspect, the present invention also relates to a use of a protein kinase inhibitor for the manufacture of a medicament for a method of treatment of cancer, wherein said cancer is characterized by one of the following:
underexpression of TTP and overexpression of HuR,
underexpression of TTP and overexpression of PLK-1,
overexpression of HuR and overexpression of PLK-1,
underexpression of TTP and overexpression of HuR and overexpression of PLK-1,
in cancer cells compared to expression in non-cancerous cells, wherein said protein kinase inhibitor is a B-Raf kinase inhibitor, VEGFR2 inhibitor, or polo-like kinase inhibitor, preferably a polo-like kinase 1 inhibitor.

In one embodiment, said protein kinase inhibitor is co-administered with a chemotherapeutic agent, and/or a checkpoint inhibitor, and/or Type-I IFN.

Said method, said cancer, said protein kinase inhibitor, said co-administered, said checkpoint inhibitor, said Type-I IFN are as defined above.

### DETAILED DESCRIPTION

In one embodiment, the present inventors disclose a protein kinase inhibitor for use in a method of treatment of cancer comprising the regulation of expression of cancer-associated ARE-containing mRNAs using a protein kinase inhibitor which is a B-Raf kinase inhibitor, VEGFR2 inhibitor or polo-like kinase inhibitor, preferably a PLK-1 inhibitor, such as volasertib, via modulation of tristetrapolin (TTP) and/or HuR. Furthermore, in one embodiment, the present invention discloses a protein kinase inhibitor for use in a method of treatment of cancer comprising administering a protein kinase inhibitor which is a B-Raf kinase inhibitor, VEGFR2 inhibitor or polo-like kinase inhibitor, preferably a PLK-1 inhibitor to reduce the half-life of ARE-containing cancer-related mRNAs, such as of uPA. The present inventors disclose that PLK-1 inhibition normalizes TTP deficiency and/or HuR overexpression in breast cancer cell lines. In addition, the present invention relates to inhibiting invasive breast cancer cell from proliferation, migration and invasion by inhibition of PLK-1 using a protein kinase inhibitor which is a B-Raf kinase inhibitor, VEGFR2 inhibitor or polo-like kinase inhibitor, preferably a volasertib. The present invention further relates to a protein kinase inhibitor for use in a method of treatment of breast cancer, in particular triple negative breast cancer, using a protein kinase inhibitor which is a B-Raf kinase inhibitor, VEGFR2 inhibitor or polo-like kinase inhibitor, preferably a PLK-1 inhibitor to normalize the TTP/ HuR ratio and to inhibit proliferation, migration, and invasion of cancer cells.

The term "cancer", as used herein, refers to a disease characterized by dysregulated cell proliferation and/or growth. The term comprises benign and malignant cancerous diseases, such as tumors, preferably, however, malignant cancerous diseases, and may refer to an invasive or non-invasive cancer. The term comprises all types of cancers, including carcinomas, sarcomas, lymphomas, germ cell tumors, and blastomas. In one embodiment, the term cancer relates to breast cancer. In one preferred embodiment, cancer relates to invasive breast cancer, more preferably triple-negative breast cancer. In one embodiment, a "tumor sample", as used herein, relates to a sample of cancerous tissue of a patient, wherein said sample may derive from a solid or a non-solid cancerous tissue. In one embodiment, said tumor sample is a sample of breast cancer, preferably invasive breast cancer, more preferably triple-negative breast cancer. A control sample or control value is used to estimate the relative expression levels of TTP, HuR, and/or PLK-1 expression in a diseased organ or tissue compared to a healthy organ or tissue.

The term "invasive breast cancer", as used herein, refers to a breast cancer that spreads beyond the layer of tissue in which it developed into surrounding healthy, normal tissue. Invasive breast cancer may spread from the breast through the blood and lymph system to other parts of the body.

The term "triple-negative breast cancer" or "TNBC", as used herein, refers to a breast cancer that does not express the genes encoding for the estrogen receptor (ER), the progesterone receptor (PR), and HER2/neu.

The term "cancer cell", as used herein, refers to a cell that exhibits abnormal proliferation and divides relentlessly, thereby forming a solid tumor or a non-solid tumor. In some embodiments of the present invention, cancer cell is used synonymously with "pathophysiological cell".

The term "non-cancer cell" or "normal cell", as used herein, refers to a cell which is not affected by aberrant expression and/or abnormal proliferation, and does not derive from cancerous tissue.

In some embodiments of the present invention, the terms "normal cell" and "non-cancer cell" are used synonymously with "physiological cell".

A "control sample", as used herein, relates to a sample comprising normal cells for determining normal expression levels in non-cancerous cells. Such a control sample may derive from the patient, wherein said control sample is taken from a healthy tissue, wherein said healthy tissue may derive from the same organ as the tumor sample of the cancerous disease, but a different site not affected by said cancerous disease, or may derive from a different organ not affected by said cancerous disease. A control sample may also relate to a sample of non-cancerous tissue of a healthy individual, or to a sample of a population of healthy individuals. In some embodiments, said control sample(s) may also relate to "control values" which reflect the normal expression levels obtained from analysis of expression in control samples, wherein said control samples derive from healthy tissue of the patient, or healthy tissue of a healthy individual, or healthy tissue of a population of healthy subjects.

The term "cancer-related genes", as used herein, refers to genes that are associated with cancerous disceases, and/or the development of cancerous diseases, and/or metastasis. In one embodiment, aberrant expression of said cancer-related genes promotes formation of a cancerous disease. For example, cancer-related genes include MMP1, MMP13, CXCR4, uPA, uPAR, IL-8, SLC2A1. In one embodiment, cancer-related genes refer to proto-oncogenes.

The term "AU-rich element" or "ARE", as used herein, refers to an adenylate-uridylate-rich element in the 3' untranslated region of a mRNA. AREs are a determinant of RNA stability, and often occur in mRNAs of proto-oncogenes, nuclear transcription factors, and cytokines. ARE-binding proteins (ARE-BP) bind to AREs and stabilize the mRNA, such as HuR, or destabilize the mRNA, such as TTP.

The term "overexpression", as used herein, refers to an elevated expression level as compared to the expression level in a non-cancer cell, referred to as "normal expression". In some embodiments, expression is compared to normal expression in a control sample, which may derive from healthy tissue of the same individual, wherein said healthy tissue may derive from a different site of the same organ as the cancerous tissue, or from a healthy individual. In some embodiments, expression is compared to normal expression in a healthy subject population. An elevated expression level may also be referred to as "increased expression level". In one embodiment, an elevated expression is an at least two-fold change in expression. The term "decreasing expression", as used herein, relates to decreasing elevated expression levels of overexpressed genes, such as HuR, PLK-1, and/or cancer-related genes, to normalize said overexpression to normal expression.

The term "underexpression", as used herein, refers to a decreased expression level as compared to the expression level in a non-cancer cell, referred to as "normal expression". In some embodiments, expression is compared to normal expression in a control sample, which may derive from healthy tissue of the same individual, wherein said healthy tissue may derive from a different site of the same organ as the cancerous tissue, or from a healthy individual. In some embodiments, expression is compared to normal expression in a healthy subject population. Said decreased expression level may also be referred to as "diminished expression level". The term "enhancing expression", as used herein, relates to increasing decreased expression levels of underexpressed genes, such as TTP, to normalize said underexpression to normal expression.

The term "normal expression" or "normal levels", as used herein, refers to expression levels in non-cancerous cells which are not affected by aberrant expression. In one embodiment, normal expression relates to expression levels of TTP, HuR, PLK-1, and/or other genes, in non-cancerous cells. In one embodiment, normal levels of TTP, HuR, PLK-1, and/or other genes, are assessed in the same subject from which the tumor sample is taken. In one embodiment, normal levels are assessed in a sample from a healthy subject. In one embodiment, normal levels are assessed in a population of healthy individuals.

The term "normalizing expression", as used herein, relates to normalizing or restoring expression levels of TTP, HuR, and/or PLK-1 to healthy, non-cancerous, normal levels, which can be achieved by administering an effective dose of a protein kinase inhibitor to a patient in need thereof having abnormal expression of TTP, HuR, and/or PLK-1. In one embodiment, said protein kinase inhibitor is a B-Raf kinase inhibitor, VEGFR2 inhibitor, or polo-like kinase inhibitor, preferably a polo-like kinase 1 inhibitor. In one embodiment, normalizing expression may relate to increasing TTP expression and/or reducing HuR expression. In one embodiment, said increase in TTP expression and/or reduction in HuR expression results in downregulation of of expression of cancer-related genes.

The term "TTP" or "tristetraprolin", as used herein, refers to a protein which binds to AU-rich elements (AREs) in the 3'-untranslated regions of ARE-containing mRNAs, and promotes degradation of said mRNAs. TTP is also known as zinc finger protein 36 homolog (ZFP36). In one embodiment, interactions of TTP and target mRNAs are affected by the phosphorylation state of TTP.

The term "HuR" or "human antigen R", as used herein, refers to a protein containing RNA-binding domains which binds to cis-acting AU-rich elements (AREs) of mRNA. Binding of HuR to an ARE of an mRNA stabilizes said mRNA. HuR post-translationally regulates gene expression by binding to and stabilizing ARE-containing mRNA. HuR levels may be elevated in cancer cells thereby increasing mRNA stability of cancer-related genes.

The term "TTP/HuR ratio", as used herein, relates to the ratio of expression levels of TTP to HuR. In one embodiment, said expression levels relate to mRNA or protein. In one embodiment, said TTP/HuR ratio is a biomarker of invasiveness or metastatic potential, i.e. the aggressiveness of cancer, in particular breast cancer. In one embodiment, a low TTP/HuR ratio indicates invasive/metastatic cancer, a high TTP/HuR ratio indicates a healthy individual or a patient with non-invasive/non-metastatic cancer. In one embodiment, a method of treatment according to the present invention is used to treat or prevent invasiveness or metastasis of cancer by increasing ("normalizing") the TTP/HuR ratio.

The term "protein kinase", as used herein, refers to an enzyme capable of phosphorylating other proteins by transferring a phosphate group from a nucleoside triphosphate to amino acids of proteins, such as serine and threonine, and/or tyrosine. Phosphorylation of proteins may result in functional modification of said proteins by changing cellular location, activity, and/or associated with other proteins. In one embodiment, a protein kinase may relate to a serine/threonine-specific protein kinase or a tyrosine-specific protein kinase, preferably a B-Raf kinase, a VEGFR2 kinase, or a polo-like kinase, more preferably a polo-like kinase 1.

The term "inhibitor", as used herein, refers to an enzyme inhibitor or receptor inhibitor which is a molecule that binds to an enzyme or receptor, and decreases and/or blocks its activity. The term may relate to a reversible or an irreversible inhibitor.

The term "protein kinase inhibitor", as used herein, refers to an inhibitor that blocks the action of one or more protein kinases. In one embodiment, said term relates to an inhibitor that attenuates the action of one or more protein kinases. In one embodiment, said protein kinase inhibitor is a serine/threonine protein kinase inhibitor, preferably a B-Raf kinase inhibitor or a polo-like kinase inhibitor, or a tyrosine kinase inhibitor, preferably a VEGFR2 inhibitor.

The term "PLK" or "polo-like kinase", as used herein, refers to a family of regulatory serine/threonine kinases of the cell cycle which plays a role in mitosis, spindle formation, meiosis, and cytokinesis. Polo-like kinase is a family of regulatory serine/threonine kinases that comprise five members including PLK-1, PLK-2, PLK-3, PLK-4, and PLK-5. They are involved in the cell cycle at different levels including mitosis, spindle formation, cytokinesis, and meiosis. PLKs share a conserved catalytic serine/threonine kinase domain at the N-terminus and a C-terminal containing two motifs called polo-boxes (polobox domain or PBD) that are involved in PLK localization, activation and binding to substrates. The N-terminals containing kinase domains are very highly conserved among all members of the family while the C-terminal carrying two polo-boxes is much less conserved among them. The N- and C domains are joining by a linker region known as the polo-box cap (Pc) that represents a part of the PBD. The PLKs are activated by upstream kinases through phosphorylation of PLK catalytic kinase domain at a short region called T-loop (containing Thr210). It has been reported that PLK-1 is phosphorylated by polo-like kinase kinase 1 (PLKK1) and protein kinase A. Also, Aurora A phosphorylates PLK-1 at Thr210 by the aid of *bora* which induces a conformation of PLK-1 priming it for Aurora-induced phosphorylation. In addition, binding of phosphorylated docking proteins to PBD leads to PLK activation. Phosphorylation of a substrate by other kinases (such as Cdk1 or Cdk5) is required to turn on PLKs activity. Absence of these phosphorylated proteins make the PBD interacts with the catalytic domain and thus inactivate PLK. Binding of phosphopeptides to PBD results in the release of the catalytic domain which converts PLK to its active form. Finally, the activity of PLKs is abolished by proteolytic degradation through the ubiquitin-proteasome pathway by the action of ubiquitin-ligase Anaphase Promoting Complex (APC) as cells exit mitosis.

The term "PLK-1" or "polo-like kinase 1", as used herein, refers to a specific kinase being a member of the family of polo-like kinases. PLK-1 is considered to be a proto-oncogene as it may be overexpressed in tumor cells. In humans, PLK-1 is expressed in the late interphase (G2) and M phases (prophase, metaphase and anaphase). During interphase and prophase, PLK-1 localizes to centrosomes, whereas at metaphase it binds to spindle poles. In the anaphase, it is distributed to the central spindle while during cytokinesis it is found in the midbody. Entry into mitosis is controlled by PLK-1, an action that is attributed to regulation of the activity of Cdk1-cyclin-B. The latter is a master regulator of M phase that is activated during G2/M transition by its dephosphorylation at ATP-binding site secondary to the action of cell division cycle25 phosphatase (Cdc25). Furthermore, chromosome segregation during anaphase and exit from mitosis are also regulated by PLK-1. This action is mediated through phosphorylation of the APC/Cyclosome (APC/C) ubiquitin ligase.

The term "PLK-1 inhibitor", as used herein, refers to an inhibitor of polo-like kinase 1. In one embodiment, said PLK-1 inhibitor is specific, i.e. said PLK-1 inhibitor only inhibits PLK-1 and does not inhibit other PLKs, such as PLK-2, PLK-3, PLK-4, and/or PLK-5, at nanomolecular concentrations. For example, volasertib is a specific PLK-1 inhibitor. In another embodiment, said term may relate to a PLK-1 inhibitor that binds to PLK-1 and that also binds to other proteins, such as other PLKs, wherein said PLK-1 inhibitor has a lower binding affinity to other proteins than to PLK-1. For example, BI-2536 is a non-specific PLK-1 inhibitor, which binds to PLK-1, and also binds to PLK2 and PLK3 at nanomolecular concentrations.

The term "administering", as used herein, refers to intravenous, oral, nasal, mucosal, intrabronchial, intrapulmonary, intradermal, subcutaneous, intramuscular, intravascular, intrathecal, intraocular, intraarticular, intranodal, intratumoral, or intrametastatical administration of a protein kinase inhibitor to a patient in need thereof.

The term "co-administering", as used herein, refers to combined administration of a protein kinase inhibitor, preferably a PLK-1 inhibitor, with one or more other substances, such as a chemotherapeutic agent, a checkpoint inhibitor, and/or Type-I IFN, and/or Type-II IFN, preferably IFNγ, and/or Type-III IFN, to a patient in need thereof.

The term "effective dose", as used herein, refers to a dose of a drug, such as a protein kinase inhibitor, which is in the range between the dose sufficient to evoke a therapeutic effect and the maximum tolerated dose. In one embodiment, a method of treatment of cancer according to the present invention comprises administering an effective dose of a protein kinase inhibitor, preferably a polo-like kinase 1 inhibitor, to a patient in need thereof. In one embodiment, a method of treatment of cancer according to the present invention comprises administering an effective dose of a protein kinase inhibitor, preferably a polo-like kinase 1 inhibitor, to a patient in need thereof, wherein said effective dose is in a dose range established for a different method of treatment comprising administering said protein kinase inhibitor, preferably a polo-like kinase 1 inhibitor, wherein said different method of treatment is for a disease, which is not characterized by one of the following: underexpression of tristetraprolin (TTP), overexpression of human antigen R (HuR), overexpression of polo-like kinase 1 (PLK-1), underexpression of TTP and overexpression of HuR, underexpression of TTP and overexpression of PLK-1, overexpression of HuR and overexpression of PLK-1, underexpression of TTP and overexpression of HuR and overexpression of PLK-1, in pathophysiological cells compared to expression in physiological cells. In one embodiment, said protein kinase inhibitor is volasertib, and said effective dose is in the range of 150 mg to 300 mg once per day to once per week.

The term "patient", as used herein, refers to a human or an animal having a cancer which is characterized by one of the following: underexpression of TTP and overexpression of HuR, underexpression of TTP and overexpression of PLK-1, overexpression of HuR and overexpression of PLK-1, underexpression of TTP and overexpression of HuR and overexpression of PLK-1, in cancer cells compared to expression in normal cells. The terms "subject" and "individual", as used herein, are used synonymously, and relate to a human or an animal.

The term "chemotherapeutic agent", as used herein, refers to a cytotoxic agent which is of use in chemotherapy of cancer. For example, a chemotherapeutic agent may relate to an alkylating agent, such as cyclophosphamide, mechlorethamine, chlorambucil, melphalan, dacarbazine, nitrosoureas, and temozolomide, or to an anthracycline, such as daunorubicin, doxorubicin, epirubicin, idarubicin, mitoxantrone, valrubicin, or to a cytoskeletal disruptor, such as paclitaxel, docetaxel, abraxane, and taxotere, or to an epothilone, or to a histone deacetylase inhibitor, such as vorinostat and romidepsin, or to an inhibitor of topoisomerase I, such as irinotecan and topotecan, or to an inhibitor of topoisomerase II, such as etoposide, teniposide, and tafluposide, or to a kinase inhibitor, such as bortezomib, erlotinib, gefitinib, imatinib, vemurafenib, and vismodegib, or to a nucleotide analogue, such as azacitidine, azathioprine, capecitabine, cytarabine, doxifluridine, fluorouracil, gemcitabine, hydroxyurea, mercaptopurine, methotrexate, and tioguanine, or to a peptide antibiotics, such as bleomycin and actinomycin, or to a platinum-based agent, such as carboplatin, cisplatin, and oxaliplatin, or to a retinoid, such as tretinoin, alitretinoin, and bexarotene, or to a vinca alkaloid derivative, such as vinblastine, vincristine, vindesine, and vinorelbine. In one embodiment, in a method of treatment of cancer according to the present invention, a chemotherapeutic agent is co-administered with said protein kinase inhibitor, wherein preferably, said chemotherapeutic agent is commonly used for the same type of cancer.

The term "checkpoint inhibitor", as used herein, refers to an agent used in cancer immunotherapy. A checkpoint inhibitor blocks an inhibitory immune checkpoint and thus restores immune system function, for example, an inhibitor of the immune checkpoint molecule CTLA-4, such as ipilimumab, or an inhibitor of PD-1, such as nivolumab or pembrolizumab, or an inhibitor of PD-L1, such as atezolizumab, avelumab, and durvalumab. In many of the embodiments, a checkpoint inhibitor relates to an antibody which targets a molecule involved in an immune checkpoint.

The term "interferon", or "IFN", as used herein, refers to a group of cytokines which are used for communication between cells and which trigger the immune system. Interferons comprise three classes which are Type-I interferons, Type-II interferons, and Type-III interferons. In one embodiment, said protein kinase inhibitor is co-administered with a Type-I IFN. In one embodiment, said protein kinase inhibitor is co-administered with a Type-II IFN, preferably IFNγ. In one embodiment, said protein kinase inhibitor is co-administered with a Type-III IFN. In one embodiment, said protein kinase inhibitor is co-administered with any combination of two or more types of interferon selected from Type-I IFN, Type-II IFN, and Type-III IFN.

The term "Type-I IFN", as used herein, relates to a large subgroup of interferons comprising IFN-α, IFN-β, IFN-ε, IFN-κ, IFN-τ, IFN-ζ, and IFN-ω.

The term "Type-II IFN", as used herein, relates to IFN-γ.
The term "Type-III IFN", as used herein, relates to IFN-λ.

The term "IFNγ", or "interferon gamma", as used herein, refers to a cytokine which is the only member of the type II class of interferons, and is an important activator of macrophages. Aberrant expression of IFNγ is associated with autoinflammatory and autoimmune diseases. IFNγ has antiviral, immunoregulatory and anti-tumor properties.

The term "post-transcriptional control" or "post-transcriptional regulation", as used herein, refers to the control of gene expression at the RNA level. The stability and distribution of different transcripts may be regulated by RNA binding proteins that control processes such as alternative splicing, nuclear degradation, processing, nuclear export, sequestration, and translation.

### BRIEF DESCRIPTION OF THE FIGURES

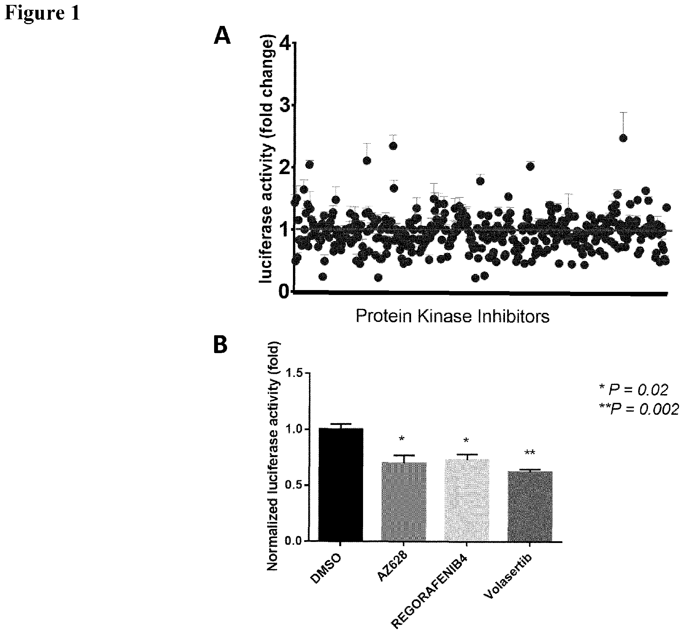

**Figure 1****:** Kinome inhibitors screening on post-transcriptional gene regulation.
   (A) Kinase inhibitor screen scatter plot: TNF-α-ARE luciferase reporter activity absolute values are plotted on the y axis against 378 corresponding kinase inhibitors on the x axis.
   (B) Luciferase activity in MDA MB231 cell line treated with DMSO (control), AZ 628, Regorafenib, and Volasertib for 24 h. Columns, mean value of experiments done in triplicate; bars, standard error of the mean (SEM), normalized luciferase activity (fold) relates to ARE/non-ARE ratio.

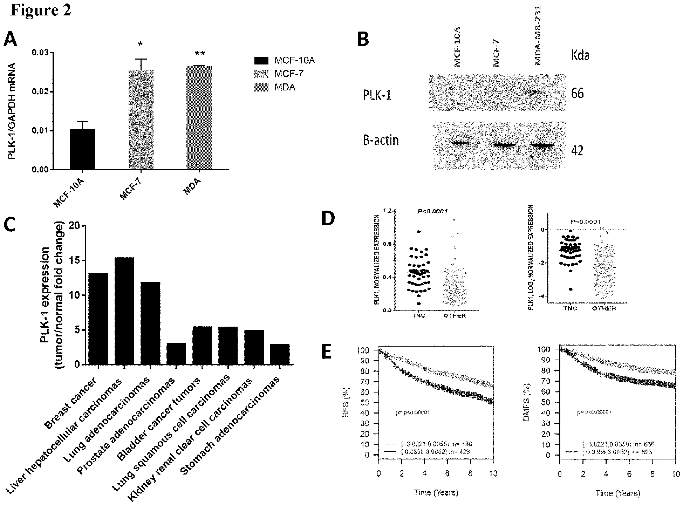

**Figure 2****:** Expression of PLK-1 in normal cells and cancer cells.
   (A) PLK-1 mRNA expression in normal cells and breast cancer cell lines quantified by RT-PCR using FAM-labelled PLK-1 and a VIC-labelled GAPDH probe.
   (B) PLK-1 protein expression in normal cells and breast cancer cells using primary antibodies for PLK-1 and beta-actin (control).
   (C) PLK-1 expression (mRNA tumor/normal fold change) in different types of cancer.
   (D) PLK-1 expression in triple negative cancer (TNC) compared to other types of breast cancer.
   (E) The effect of PLK-1 overexpression on the 10-years relapse-free survival (RFS) and distant metastasis-free survival (DMFS) of cancer patients. Values are shown as mean ± standard error of the mean (SEM), and comparison was performed using Student's t-test, wherein **P* ≥ 0.01, ***P* ≥ 0.001.

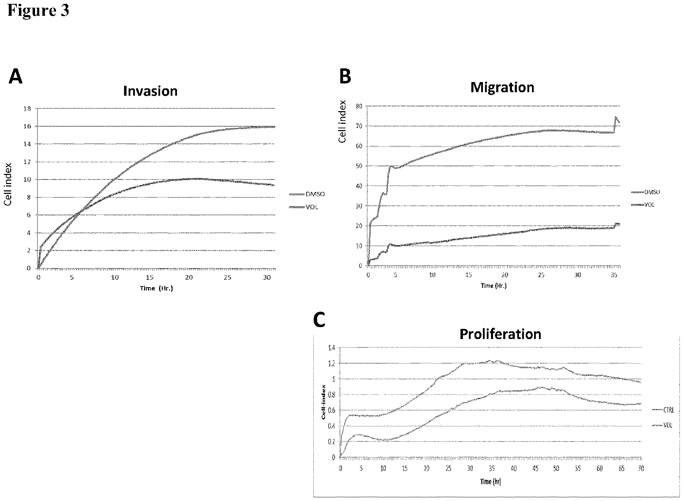

**Figure 3****:** The effect of volasertib on MDA-MB-231 behavior.
   (A)MDA-MB 231 cells were treated with DMSO and volasertib for 24 h. Then, cell invasion was monitored continuously over 30 hours using RTCA Software.
   (B) The same protocol was repeated without applying Matrigel to measure cellular migration.
   (C) MDA-MB-231 proliferation was monitored for 70 hours after treatment with 300 nM volasertib.
**Figure 4****:** Effect of volasertib on the expression of various cancer-related genes having ARE-containing mRNAs. mRNA expression of IL-8, uPA, uPAR, SLC2A1, CXCR4, MMP13 is shown for control cells and volasertib treated MDA-MB-231 cells. Values are shown as mean ± SEM and statistical analysis was performed using Student's t-test.
**Figure 5****:** Effect of volasertib on TTP expression and activity. MDA-MB 231 cells were treated with DMSO or volasertib for 24 h.
   (A) Effect of volasertib on *TTP* and (B) *HuR* mRNA expression; qPCR for *TTP* and *HuR* was performed: *****p*< 0.001.
   (C) mRNA decay curve for *uPA* in MDA-MB-231 cells using the one-phase exponential decay model.
   (D) The correlation between PLK-1 and TTP expression in cancer obtained from TCGA data, using the Oncomine portal. Values were expressed as mean ± SEM and comparison was performed using Student's t-test.

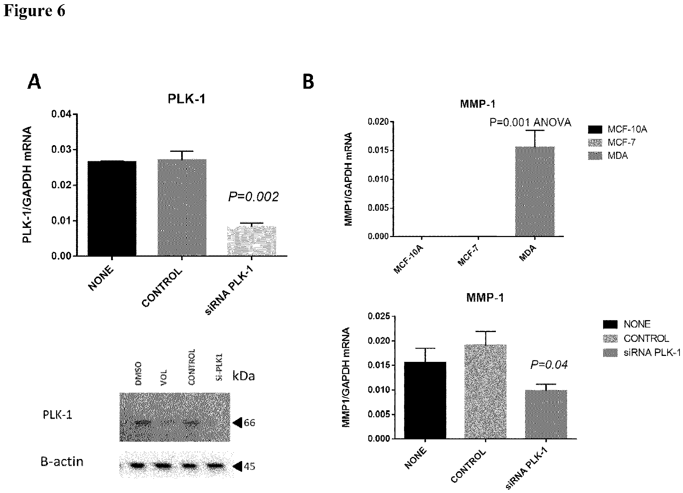

**Figure 6****:** Knockdown PLK-1 using siRNA in MDA-MB-231.
   (A) Expression of PLK-1 mRNA (upper panel) and protein (lower panel) after siRNA treatment.
   (B) Expression of MMP1 in normal cells (MCF-10A, non-tumorigenic cell line) and cancer cells (MCF-7, hormone responsive breast cancer cell line; and MDA, triple-negative breast cancer cell line) as shown in the upper panel, and after gene silencing of PLK-1 using siRNA (lower panel). Values are shown as mean ± SEM, and comparison was performed using Student's t-test.

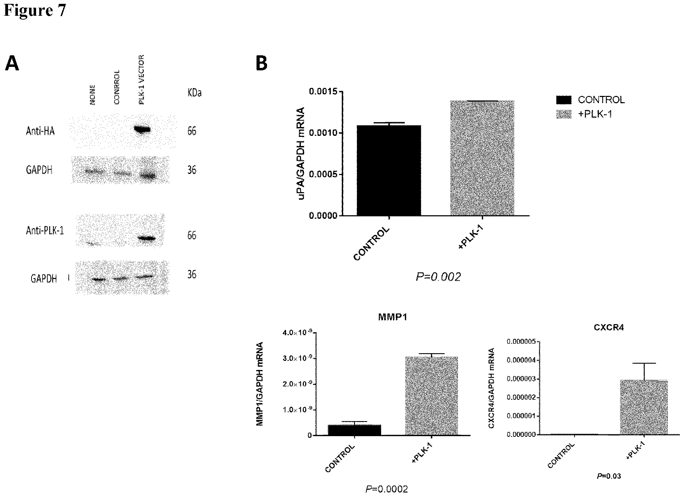

**Figure 7****.** PLK-1 overexpression in MCF10A Cells.
   (A) Expression of PLK-1 protein after transfection with PLK-1-vector.
   (B) mRNA expression of uPA, MMP1 and CXCR4. Values are shown as mean ± SEM, and comparison was performed using Student's t-test. +PLK-1 refers to PLK-1 overexpression; CXCR4 to chemokine receptor-4 (CXCR4); MMP1 to matrix metalloproteinase 1, and uPA to urokinase plasminogen activator.

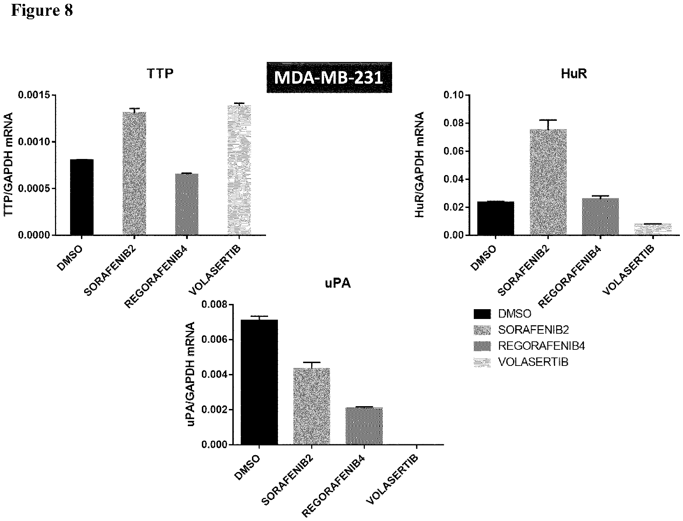

**Figure 8****.** Effect of protein kinase inhibitors sorafenib2, regorafenib4, and volasertib on expression of TTP, HuR, and uPA in MDA-MB-231 cells.

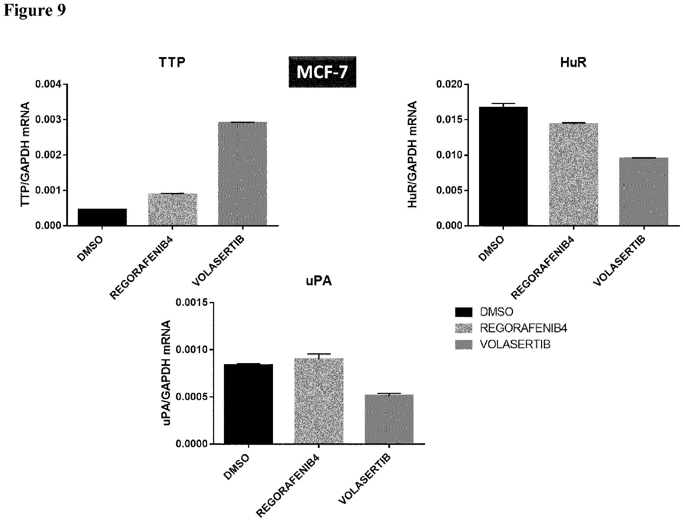

**Figure 9****.** Effect of protein kinase inhibitors regorafenib4 and volasertib on expression of TTP, HuR, and uPA in MCF-7 cells.

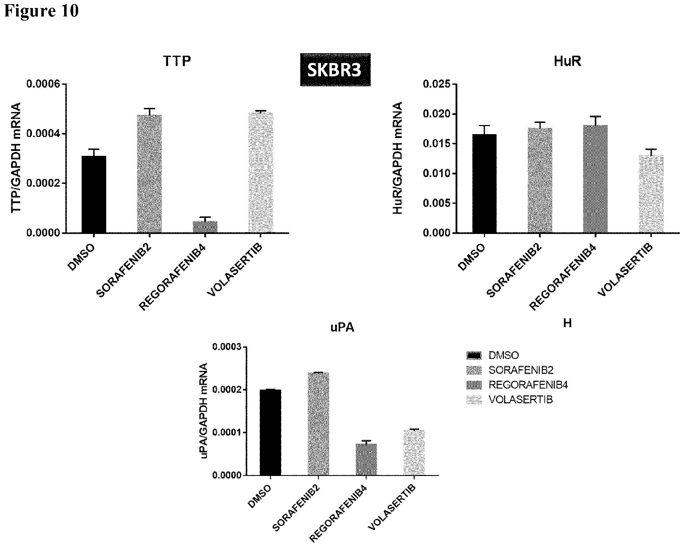

**Figure 10****.** Effect of protein kinase inhibitors sorafenib2, regorafenib4, and volasertib on expression of TTP, HuR, and uPA in SKBR3 cells.

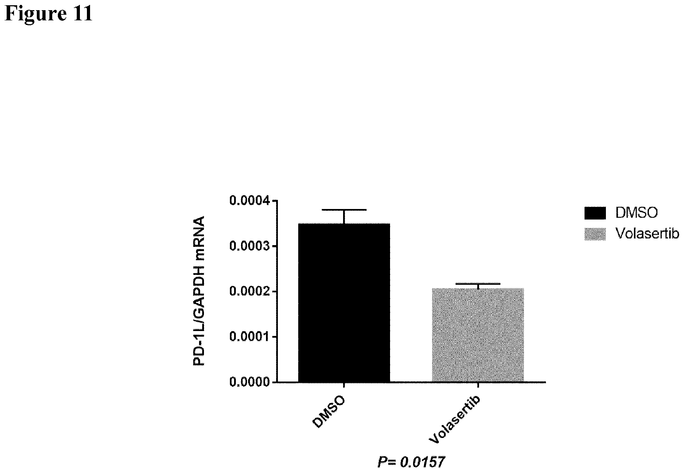

**Figure 11****.** Volasertib reduces PD-1L expression.

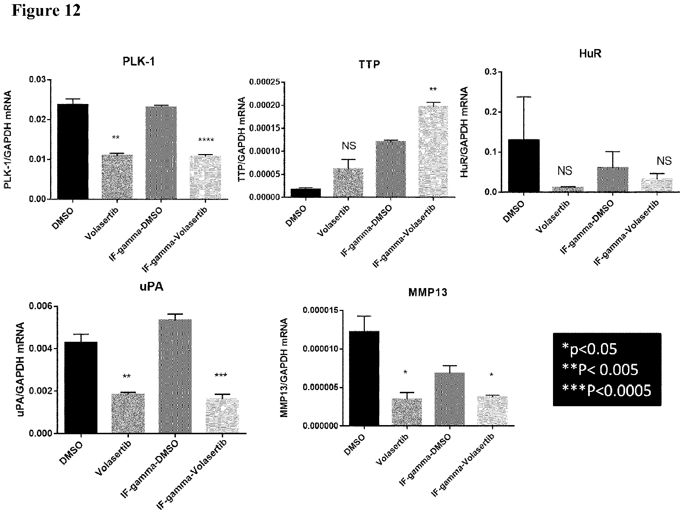

**Figure 12****.** Effect of volasertib and IFNγ on expression of PLK-1, TTP, HuR, uPA, and MMP13.

### EXAMPLES

### Example 1: Kinome inhibitors screening on post-transcriptional gene regulation.

### Cell lines

Breast cancer cell lines MDA-MB-231, SKBR3, and MCF-7, and the normal-like breast cell line MCF10A were obtained from American Type Culture Collection (ATCC, Rockville, MD, USA). MDA-MB-231 and MCF-7 cell lines were cultured in Dulbecco's modified Eagle's medium (DMEM; Invitrogen, Carlsbad, CA, USA) at 37 °C supplemented with 2 mM glutamine and 10 % fetal bovine serum (FBS). SKBR3 cells were grown in McCoy's 5a Medium (McCoy's 5A; Thermo Fisher Scientific, Waltham, MA, USA) with 10 % FBS. MCF10A were maintained in Ham's F12-DMEM mixture (DMEM/F12 Ham; Thermo Fisher Scientific, Waltham, MA, USA) and supplemented with 20 ng/ml epidermal growth factor (EGF), 0.01 mg/ml bovine insulin and 500 ng/ml hydrocortisone (Sigma, St. Louis, MO, USA). All culture media were supplemented with 1 % penicillin-streptomycin antibiotics (Sigma, St. Louis, MO, USA). All transfections were performed in reduced serum media using Lipofectamine 2000 (Invitrogen).

### Reporter plasmids

Reporters were designed to be driven by the ribosomal protein subunit 30 (RPS30) promoter. The promoter was amplified using PCR with primers specific to the flanking region of the *RPS30* promoter sequence. The gene structure of RPS30 was obtained from the Ribosomal Protein Database (http://ribosome.med.miyazaki-u.ac.jp). The forward and reverse primers included the restriction sites, *EcoRV* and *SalI* sites. Amplified DNA fragments were then resolved in 1.2 %-1.5 % agarose gel and the amplicon bands were excised, purified and cloned into the promoterless plasmid. TNF-α 3'-UTR and TNF-α ARE were used to study the role of AU-rich elements in the action of PKs inhibitors. The AU-rich sequence that comprised 250 bases from TNF-α 3'UTR (1200-1450 bp, NM_00059) was amplified by PCR using the forward primer 5'-CAGCAGGATCCAGAATGCTGCAGGACTTGAG-3' (SEQ ID NO:1) and the reverse primer 5'-CGACCTCTAGACTATTGTTCAGCTCCGTTT-3' (SEQ ID NO:2). The TNF-α-ARE sequence was made by annealing two synthetic complementary oligonucleotides of 70 bases that correspond to TNF-α ARE. The control reporter was constructed to have the basic structure of post-transcriptional reporter except 3'UTR which lack the AU-rich element sequence like this of bovine growth hormone.

### Protein kinase inhibitors library

Selleckchem Kinase Inhibitor Library that includes a selection of 378 pharmacologically active inhibitors of a number of protein kinases was purchased from Selleckchem. (Houston, TX, USA). This library is a collection of 378 kinase inhibitors some of which have been approved by the FDA. Each compound is provided as a pre-dissolved 10 mM dimethyl sulfoxide (DMSO) solution as a 96 well format tube (100 µL). The compounds were diluted by OPTI-MEM (Thermo Fisher Scientific, Waltham, MA, USA) to a final concentration of 5 µM.

### Reporter assay

Post-transcriptional and control reporter constructs were transfected to MDA-MB-231 cells separately that were seeded in 96-well microplates at a density of 4 × 10⁴ cells/well and incubated overnight. Using lipofectamine 2000 protocol (Invitrogen, Carlsbad, CA, USA), the cells were transfected with 10 ng of either RPS30-luciferase-control 3'UTR or RPS30-luciferase-ARE 3'UTR reporter plasmids. After 24 h, the cells were treated with each member of protein kinase inhibitors kit for 24 h. Then, luciferase reaction was performed as prescribed by manufacture's protocol (Nano-Glo Luciferase, Promega, Madison, WI, USA)/well. After 15 minutes, the reporters' activities were measured through the measurement of the chemiluminescence (Figure 1) after treatment using a Zenith 3100 (Anthos Labtec, Eugendorf, Austria).

### Statistical analysis

Data are presented as means ± standard error of the mean (SEM). Two-sample Student's *t*-test was used to determine the differences between two data sets. One-way analysis of variance (ANOVA) was used to compare three or more data columns. Two-way analysis of variance was used to analyze two groups of data, each having two data columns. Analysis was performed using GraphPad Prism version 6.00 for Windows, (GraphPad Software, La Jolla California, USA). For high throughput screening (HTS) hit selection (protein kinase inhibitors), strictly standardized mean difference (SSMD) test was used. This statistical test measures the size of effect of each member in a group relative to the other members. It is the mean of each member divided by the standard deviation of the difference between two random values from different groups. Based on the value of SSMD, the effect of each member of the library was classified as strong (*β* ≥ 5), moderate (1≤ *β* <5), and weak (*β* < 1). In this study, only drug with SSMD score more than or equal to 5 or less than or equal to -5, i.e. strong *β*, where selected in the primary screening.

### PLK-1 regulates ARE-mediated post-transcriptional pathways in breast cancer

MDA-MB-231 cells are a model for highly invasive breast carcinomas and characterized by aberration in several protein kinase pathways including ERK, PI3K, MAP kinases, Ras, and many receptor and nonreceptor tyrosine kinases. To perform a functional kinome screen, the PK inhibitors were examined for their effect on the expression of ARE and non-ARE containing reporter using optimized and highly selective post-transcriptional reporter system as set forth above. The screening was performed in three stages to detect the protein kinase inhibitors that potentially affect the phosphorylation of an ARE-BP, as shown by lowering of the expression of ARE-containing reporter activity. In the primary screening, 87 out of 378 drugs in the PK library were found to reduce the expression of ARE-containing reporter without comparing their effect to a control reporter. Only three of the protein kinase inhibitors were confirmed to be involved in ARE-dependent mRNA regulation in the later stages of screening when they were compared to a non-ARE reporter. These potent protein kinase inhibitors were AZ628, Regorafenib, and Volasertib, and their substrates are Raf, VEGFR2 and PLK-1 respectively (Figure 1B).

First, a "primary screening" was performed in which MDA-MB-231 cells were transfected with the post-transcriptional luciferase reporter with 3'UTR-containing ARE and then treated with 5 µM/well of each member in the PK inhibitor library or DMSO as a vehicle control for 16 hr (Figure 1A). Finally, the drugs that specifically reduced the expression of the ARE reporter in comparison to the non-ARE reporter were selected in a second stage which aimed at narrowing the primary screen results to eliminate non-specific effects on non-ARE reporter activity, and these were subjected to further investigation. In this stage, the cells were transfected with both the ARE and non-ARE control post-transcriptional reporters and treated with different doses of the protein kinase inhibitors (0.5, 2, and 5 µM concentrations). Three drug groups were discovered that reduce ARE-post-transcriptional reporter activity (Figure 1B), namely rapidly accelerated fibrosarcoma kinases (B-Raf), VEGF receptors type 2 (VEGFR2), and polo-like kinase 1 (PLK-1), comprising AZ 628 (pan-Raf inhibitor), Regorafenib (BAY 73-4506), inhibiting Raf-1 and VEGFR1-3, and Volasertib (BI 6727, PLK-1 inhibitor).

### Example 2: Expression of PLK-1 in normal cells and cancer cells.

Methods were performed as described in the foregoing example.

### Plasmids and RNA interference

Vector used for *PLK-1* overexpression was obtained from Genecopoeia (Rockville, Maryland, United States) and had been designed to have human influenza hemagglutinin (HA) tag. RNA interference studies were performed using chemically synthesized siRNA duplexes purchased from Santa Cruz (Santa Cruz Biotech, CA) for silencing of *PLK-1* and a control siRNA. Western blotting and RT-PCR were utilized to determine the efficiency of siRNA silencing after 48 hr. Lipofectamine LTX was used for siRNAs transfection following the manufacturer protocol (Invitrogen, Carlsbad, CA, USA). The final concentration of siRNAs used for transfection was 50 nM.

### PLK-1 is overexpressed in cancer cells

The expression of PLK-1 in normal MCF-10A and two breast cancer cell lines, MDA-MB-231 and MCF-7, was measured using RT-PCR. As shown in Figure 2, breast cancer cells exhibited moderate to high expression of PLK-1 compared to normal cell line. The MDA-MB-231 cells significantly express higher levels of PLK-1 mRNA than normal breast epithelial cells. Concurrently, the triple negative breast cancer subtype is characterized by higher expression of PLK-1 compared to the other types of breast cancer according to data obtained from TCGA data (Figure 2D), using the Oncomine portal. Patients' data also revealed that PLK-1 is overexpressed in a wide range of human cancers including breast, liver, lung, prostate, kidney, gastric and bladder carcinomas (Figure 2C). High expression of PLK-1 was found to be associated with reduced survival among cancer patients. (Figure 2E).

### Example 3: PLK-1 inhibitor (Volasertib) reduces the expression of ARE-containing cancer genes

Methods were performed as described in the foregoing examples.

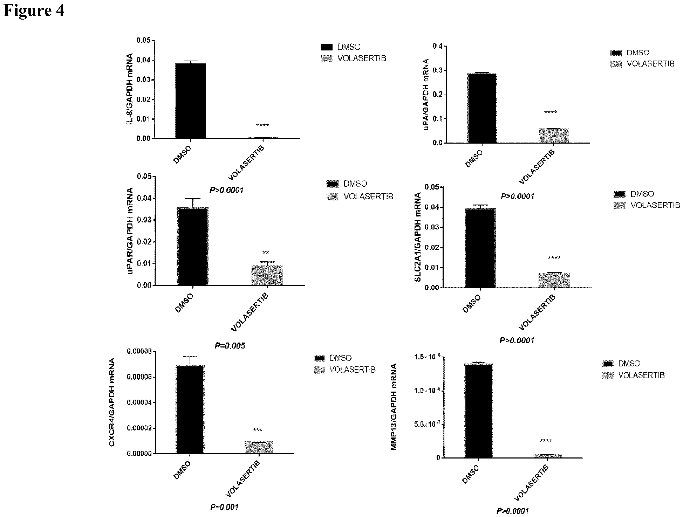

The effect of volasertib was studied on the expression of known genes that have been shown to be upregulated in cancer and bearing ARE sequence in their mRNA. These include IL-8, solute carrier family 2 member 1 (SLC2A1), uPA, uPAR, CXCR4, MMP13, and HuR. The MDA-MB-231 cells were treated with 330 nM volasertib or DMSO for 24 hr, then quantitative RT-qPCR was performed in using FAM-labelled TaqMan probe of the above genes and normalized to GADPH (Figure 4). As can be observed from Figure 4, the expression of all ARE-containing cancer genes was significantly reduced using volasertib.

### mRNA half-life and quantitative reverse transcription-polymerase chain reaction

Cells were cultured in six-well plates and either treated with DMSO or drugs for 24 h. Total RNA was then extracted using Trizol reagent (TRI Reagent, Sigma-Aldrich, St Louis, MO). The cells were lysed directly on the culture dish by adding 1 ml of the TRI Reagent per 10 cm² surface area. Reverse transcription for preparation of cDNA was performed using 3 µg of total RNA, 150 ng random primers, 0.1 M dithiothreitol (DTT), 10 mM deoxynucleotide triphosphate (dNTP) and 200 U of SuperScript II (Invitrogen, Foster City, CA). The quantitative RT-QPCR was performed in multiplex in the Chroma 4 DNA Engine cycler (BioRad, Hercules, CA, USA) using FAM-labelled TaqMan probes (Applied Biosystems, Foster City, CA, USA) for TTP (*ZFP36*), HuR (*ELAVL1*), uPA (*PLAU*), ,CXCR4, MMP-1, MMP-13, IL-8, PLK-1 while a VIC-labelled glyceraldehyde-3-phosphate dehydrogenase (*GAPDH)* probe was used as the endogenous control. Samples were amplified in triplicate and quantification of relative expression was performed using the estimation of quantitation cycle (Cq) method.

For half-life experiments, 5 µg/ml of Actinomycin D (ActD; Sigma-Aldrich, St Louis, MO) was added to the cells for 1, 2, 4 and 6 h prior to extraction of total RNA using Trizol. The reverse transcription reaction and quantitative PCR were performed as described above. The half-life of mRNAs was estimated using the one-phase exponential decay method using GraphPad Prism software (GraphPad Software, San Diego, CA).

### Western blotting

The cells were lysed in a mixture of 2 x Laemmli buffer (BioRad, Hercules, CA, USA) and DTT (1:1). The cell lysates were loaded and subjected to electrophoresis on 4-12 % NuPAGE Bis-Tris gel (Invitrogen, Foster City, CA, USA). Rainbow protein molecular weight marker was used as a ladder to detect the size of the proteins. Then, the proteins were transferred from the gel to nitrocellulose membranes (Hybond ECL; Amersham Biosciences, Piscataway, NJ) in the presence of NuGAGE 20 x transfer buffer (Invitrogen, Foster City, CA, USA). After blocking, membranes were incubated with primary antibodies diluted in 5 % bovine serum albumin (BSA) (Sigma-Aldrich, St Louis, MO) 4 °C overnight. Antibodies used include; mouse anti-TTP (dilution 1:1000, Santa Cruz Biotech, Santa Cruz, CA), rabbit anti-PLK-1, rabbit anti-caspase 3, rabbit anti-Bcl2, rabbit anti-actin (dilution 1:1000, Cell signaling, Massachusetts, USA), rabbit anti- AUF1, anti-KSRP (dilution 1:500, Abcam, MA, USA), anti-HA (Human influenza hemagglutinin (HA); dilution 1:5000, Roche, Upper Bavaria, Germany). Then after, the membranes were incubated with enzyme (e.g. horseradish peroxidase, HRP) conjugated with goat anti-rabbit or anti-mouse secondary antibodies (diluted in 5 % BSA, 1:2000 dilution) (Santa Cruz Biotech, Santa Cruz, CA) for 1-3 hr. Protein bands were detected using ECL Western blotting detection reagents (Amersham Biosciences, Amersham, UK) in Molecular Imager ChemiDoc machine (BioRad, Hercules, CA, USA).

### Example 4: Invasion, migration, and proliferation of cells

Methods were performed as described in the foregoing examples.

### Invasion and migration assays

MDA-MB-231 cells were seeded in 6-well cell culture plates and incubated overnight. The cells were treated with the selected protein kinase inhibitors and incubated overnight. Then they were reseeded onto the invasion chamber in serum-free media at a density of 2 x 10⁴ cells per well using 16-well CIM-Plate in Real-Time Cell Analysis (RTCA) Dual Plate (DP) Analyzer (ACEA Bioscinece, California, USA) that was loaded inside the incubator. The lower chambers to which cells will migrate were prepared to contain chemoattractant consisted of 10 % FBS. For invasion assays, Matrigel (BioCoat, BD Biosciences, MA) which resembles the complex extracellular environment found in many tissues was prepared and used to coat the upper chamber of CIM plates. Invasion and migration were monitored continuously over 72-hour period using RTCA Software (ACEA Bioscinece, California, USA). The RTCA Instrument automatically monitors the cells every 15 minutes for 100 repetitions. For proliferation assays, the same principle was applied using a single chamber containing complete DMEM media (Figure 3).

### Volasertib inhibits invasion, migration and proliferation of breast cancer cells

Based on the dose-response curve, 330 nM was selected to be used as a standard dose for the experiments. In the invasion assay, MDA-MB-231 cells were treated with volasertib or DMSO for 24 hr, then invasion was monitored for 30 hr. As shown in (Figure 3A), MDA-MB-231 cells showed a reduced invasive behavior after treatment with volasertib compared to the control DMSO and the effect started as early as 6 hr after treatment. Similarly, volasertib was shown to reduce breast cancer cells migration (Figure 3B) for thirty hours after treatment. Proliferation of MDA-MB-231 was found to be significantly reduced after treatment with volasertib (300 nM) compared to DMSO (Figure 3C).

### Example 5: Correlation of PLK-1 and TTP expression

Methods were performed as described in the foregoing examples.

### Patient data and analysis

The Cancer Genome Atlas (TCGA) was searched using the Oncomine web portal, www.oncomine.com. TCGA is collaboration between the National Cancer Institute (NCI) and National Human Genome Research Institute (NHGRI) that provides maps for the genomic changes in different types of cancer. According TCGA, they have declared the following: 'All samples in TCGA have been collected and utilized following strict human subjects protection guidelines, informed consent'.

### Volasertib increases TTP expression and reduces HuR

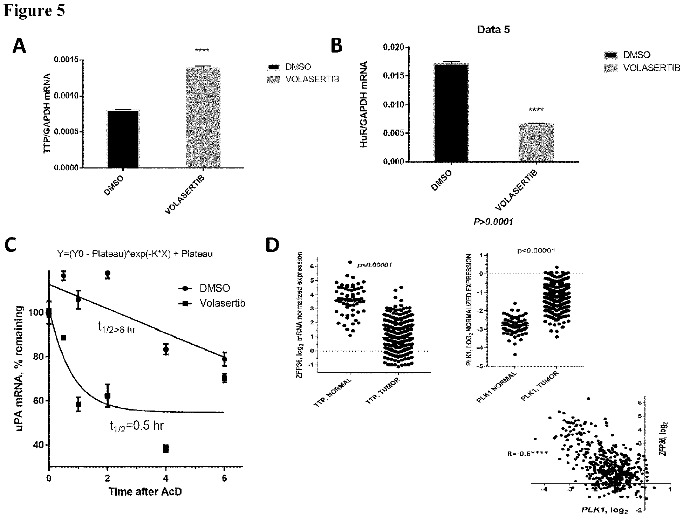

PLK-1 inhibition using volasertib in MDA-MB-231 cells resulted in 40% enhancement of *TTP* and 60% reduction of *HuR* mRNA expressions (Figure 5A,B). To study the influence of PLK-1 inhibition on ARE-containing mRNA stability, the effect of volasertib treatment on the half-life of uPA was analyzed. Half-life of uPA mRNA was reduced from > 6 hr to 0.5 hr in response to volasertib treatment compared to the DMSO control (Figure 5C). The relationship between PLK-1 and TTP expression in normal and cancer patients was analyzed and as shown in Figure 5D, patients having a TNBC tumor have high PLK-1 and low TTP levels. Compatible with the findings of the inventors, the higher the level of PLK-1, the lower the TTP expression.

### PLK-1 silencing reduces the expression of TTP targets

To investigate whether the effects produced by volasertib are attributed to PLK-1 inhibition specifically, siRNA was used to knock-down PLK-1 in MDA-MB-231 cells. After transfection, the gene expression and protein level of PLK-1 were monitored to ensure the efficiency of PLK-1 siRNA (Figure 6A). The influence of PLK-1 silencing on ARE-BPs action was verified by measuring one of three ARE-bearing targets, namely MMP-1. Expression of MMP-1 was found to be significantly high in MDA-MB-231 cell line and nearly undetectable in normal MCF-10A and cancerous MCF-7 cells (Figure 6B, upper panel). Treatment with PLK-1 siRNA significantly lowered the level of MMP-1 mRNA compared to the control siRNA in MDA-MB-231 cells (Figure 6B, lower panel).

### PLK-1 overexpression inhibits the action of TTP

For further characterization of the role of PLK-1 in ARE-mediated regulation of post-transcription, we overexpressed PLK-1 in normal MCF10A breast cells and measured the expression of some ARE-containing genes. Overexpression of PLK-1 in MCF10A cells was verified by western blotting (Figure 7A). As shown in Figure 7B, the overexpression of PLK-1 in MCF10A cell was associated with moderate increase in uPA mRNA expression. As expected, the expression of MMP1 and CXCR4 was very low in normal cells, yet PLK-1 overexpression increased their levels.

The present inventors disclose a method of treatment of cancer using ARE-mediated gene post-transcriptional regulation involving a protein kinase, preferably polo-like kinase 1 (PLK-1). PLK-1 inhibitors volasertib and BI 2536 reduced the TNF-ARE luciferase reporter activity by 40 % and 35 %, respectively. With regard to volasertib, the same reduction in percent reporter activity was observed in the secondary screening compared to the control reporter, wherein with regard to BI 2536, the reduction in percent reporter activity was reduced to 16 % in the secondary screening. Other PLK inhibitors including rigosertib, HMN-214, GSK461364, Ro3280 and NMS-P937 did not reduce ARE-reporter activity. Rigosertib is a PLK-1 inhibitor but shows more than 30-fold selectivity against PLK-2 with no activity on PLK-3 whereas GSK461364 and NMS-P937 are PLK-1 inhibitors in phase 1 trial with more than 1000-fold activity against PLK2 and PLK-3.

The present inventors disclose that triple negative breast cancer cells express significantly higher levels of PLK-1 mRNA and protein compared to HER negative cancer and normal breast epithelial cells. The present inventors further disclose that the effect of PLK-1 inhibition is consistent among different types of breast cancer including triple-negative, HER negative, and ER-PR negative breast cancers, as shown through the actions induced by PLK-1 inhibitor volasertib on MDA-MB-231, MCF7, and SKBR3 cell lines, namely increasing the expression of TTP, decreasing the HuR level, and downregulating the TTP target uPA. The present inventors disclose that triple-negative breast cancer cell proliferation is significantly reduced by volasertib. The present inventors further disclose that invasion and migration of MDA-MB-231 were significantly reduced and cancer-related genes, such as CXCR4, MMP1, MMP13, uPA, and uPAR, were downregulated upon PLK-1 inhibition. Accordingly, the present inventors disclose that a protein kinase inhibitor, preferably a PLK-1 inhibitor, more preferably a specific PLK-1 inhibitor post-transcriptionally regulates expression of cancer-related genes, and a method of treatment of cancer comprising administering said protein kinase inhibitor, preferably a PLK-1 inhibitor, more preferably a specific PLK-1 inhibitor, to a patient in need thereof.

The present inventors further disclose that PLK-1 inhibition reduces the half-life of TTP target uPA, and that PLK-1 inhibitor volasertib reduces expression of ARE-containing mRNA targets, such as CXCR4, IL-8, MMP1, MMP13, uPA, uPAR, and SLC2A1 mRNA. In addition, the present invention discloses that volasertib increases the TTP level and reduces the HuR level. Correspondingly, PLK-1 overexpression resulted in increasing the expression of TTP targets, such as uPA, MMP1, and CXCR4.

The present invention discloses the effect of a wide range of PKs on ARE-mediated regulation of gene expression, in particular three kinase pathways are disclosed to be involved in ARE-dependent mRNA regulation, including Raf, VEGFR2 and PLK-1. PLK-1 is disclosed to regulate the expression of many ARE-containing mRNAs especially those involved in cancer by phosphorylation of ARE-BPs. PLK-1 inhibitor volasertib is disclosed to reduce the level and activity of an ARE-containing reporter and to reduce the half-life of ARE-containing uPA mRNA. PLK-1 inhibition is disclosed to normalize TTP deficiency and HuR overexpression in breast cancer, and inhibited invasive breast cancer cell proliferation, migration and invasion. The present invention discloses a method of treatment of cancer comprising PLK-1 inhibition, wherein PLK-1 inhibition normalizes the TTP/ HuR ratio and inhibits cancer cell proliferation, migration and invasion.

### REFERENCES

[1] Bhola NE, Jansen VM, Bafna S, Giltnane JM, Balko JM, et al. (2014) Kinome-wide functional screen identifies role of PLK1 in hormone-independent, ER-positive breast cancer. Cancer Research - Therapeutics, Targets, and Chemical Biology. DOI: 10.1158/0008-5472.CAN-14-2475.
[2] Maire V, Némati F, Richardson M, Vincent-Salomon A, Tesson B, et al. (2012) Cancer Research - Therapeutics, Targets, and Chemical Biology. DOI: 10.1158/0008-5472.CAN-12-2633.

The features of the present invention disclosed in the specification, the claims, and/or in the accompanying figures may, both separately and in any combination thereof, be material for realizing the invention in various forms thereof.

## Claims

1. A protein kinase inhibitor for use in a method of treatment of cancer in a patient, wherein said cancer is **characterized by** one of the following:
underexpression of TTP and overexpression of HuR,
underexpression of TTP and overexpression of PLK-1,
overexpression of HuR and overexpression of PLK-1,
underexpression of TTP and overexpression of HuR and overexpression of PLK-1, in cancer cells compared to expression in non-cancerous cells;
said method comprising administering an effective dose of a protein kinase inhibitor to a patient in need thereof having said cancer, wherein said protein kinase inhibitor is a B-Raf kinase inhibitor, VEGFR2 inhibitor, or polo-like kinase inhibitor, preferably a polo-like kinase 1 inhibitor.

2. The protein kinase inhibitor for use according to claim 1, wherein said method comprises the steps of:
a. Receiving a sample of a tumor (tumor sample), and optionally a control sample, from the patient,
b. Determining the level of expression of TTP, and/or HuR, and/or PLK-1 in said tumor sample, and optionally in said control sample,
c. Administering a therapeutically effective amount of said protein kinase inhibitor, preferably of said polo-like kinase inhibitor (PLK), more preferably a PLK-1 inhibitor to the patient, if there is a reduced expression of TTP and/or increased expression of HuR, and/or increased expression of PLK-1 in the tumor sample as compared to a control sample, which is optionally the control sample of said patient, as determined in step b).

3. The protein kinase inhibitor for use according to any of claims 1-2, wherein said cancer comprises cells having diminished levels of TTP and/or elevated levels of HuR compared to normal cells.

4. The protein kinase inhibitor for use according to any of the foregoing claims, wherein said cancer is invasive breast cancer.

5. The protein kinase inhibitor for use according to any of the foregoing claims, wherein said cancer is triple-negative breast cancer.

6. The protein kinase inhibitor for use according to any of the foregoing claims, wherein said protein kinase inhibitor is selected from the group comprising AZ628, sorafenib2, TAK-6323, regorafenib4, CEP-32496, cabozantinib, and polo-like kinase inhibitors including volasertib.

7. The protein kinase inhibitor for use according to any of the foregoing claims, wherein said protein kinase inhibitor is a polo-like kinase inhibitor, preferably a polo-like kinase 1 inhibitor, preferably a specific polo-like kinase 1 inhibitor.

8. The protein kinase inhibitor for use according to claim 7, wherein said polo-like kinase inhibitor is selected from the group comprising PCM-075, volasertib, BI 2536, rigosertib (ON 01910), HMN-214, GSK461364, Ro3280, NMS-P937, TAK-960, cyclapolin 1, DAP-81, ZK-thiazolidinone, compound 36 (imidazopyridine derivative), LFM-A13, poloxin (thymoquinone derivative), poloxipan, purpurogallin (benzotropolone-containing compound), MLN0905, SBE13.

9. The protein kinase inhibitor for use according to claim 7, wherein said polo-like kinase inhibitor is a polo-like kinase 1 inhibitor.

10. The protein kinase inhibitor for use according to claim 7, wherein said polo-like kinase inhibitor is a dihydropteridinone-based derivative, preferably volasertib.

11. The protein kinase inhibitor for use according to any of the foregoing claims, wherein said protein kinase inhibitor is co-administered with a chemotherapeutic agent, and/or a checkpoint inhibitor, and/or an interferon selected from Type-I IFN, Type-II IFN and Type-III IFN.

12. The protein kinase inhibitor for use according to claim 11, wherein said checkpoint inhibitor is selected from CTLA-4, PD-1, and PD-L1 targeting agents.

13. The protein kinase inhibitor for use according to claim 11 or 12, wherein said checkpoint inhibitor is selected from the group comprising ipilimumab, tremelimumab, nivolumab, MK-3475, MPDL-3280A, MEDI-4736, and BMS-936559.

14. The protein kinase inhibitor for use according to claim 11, wherein said interferon, preferably said Type-I IFN and/or said Type-II IFN and/or said Type-III IFN, enhances TTP expression and/or reduces HuR expression, wherein preferably said protein kinase inhibitor and said interferon synergistically enhance TTP expression and/or reduce HuR expression.

15. The protein kinase inhibitor for use according to any of the foregoing claims, wherein said TTP expression is increased and/or HuR expression is decreased by administering said protein kinase inhibitor.

16. The protein kinase inhibitor for use according any of the foregoing claims, wherein cancer-related genes are post-transcriptionally controlled by administering said protein kinase inhibitor.

17. A protein kinase inhibitor for use in a method of post-transcriptional control of cancer-related genes comprising administering an effective amount of a protein kinase inhibitor to a subject in need thereof, wherein said protein kinase inhibitor is a B-Raf kinase inhibitor, VEGFR2 inhibitor, or polo-like kinase inhibitor, preferably a polo-like kinase 1 inhibitor.

18. The protein kinase inhibitor for use according to claim 17, wherein said administering of a protein kinase inhibitor results in the reduction of expression of a mRNA comprising an AU-rich element.

19. The protein kinase inhibitor for use according to claim 17 or 18, wherein said protein kinase inhibitor is selected from inhibitors of polo-like kinase 1, including AZ628, sorafenib2, TAK-6323, regorafenib4, CEP-32496, cabozantinib, PCM-075, volasertib, BI 2536, rigosertib (ON 01910), HMN-214, GSK461364, Ro3280, NMS-P937, TAK-960, cyclapolin 1, DAP-81, ZK-thiazolidinone, compound 36 (imidazopyridine derivative), LFM-A13, poloxin (thymoquinone derivative), poloxipan, purpurogallin (benzotropolone-containing compound), MLN0905, SBE13, wherein said protein kinase inhibitor is preferably a polo-like kinase inhibitor, more preferably a polo-like kinase 1 inhibitor, and more preferably volasertib.


SEQUENCE LISTING

<110> King Faisal Specialist Hospital & Research Centre

<120> Protein kinase inhibitors and their uses in the treatment of cancer

<130> K31726EP

<150> US16007146
<151> 2018-06-13

<160> 2

<170> BiSSAP 1.3.6

<210> 1
<211> 31
<212> DNA
<213> Artificial Sequence

<220>
<223> Forward primer

<400> 1
cagcaggatc cagaatgctg caggacttga g                                    31

<210> 2
<211> 30
<212> DNA
<213> Artificial Sequence

<220>
<223> Reverse primer

<400> 2
cgacctctag actattgttc agctccgttt                                      30